# EUROPEAN PATENT SPECIFICATION

(11) **EP 1 879 598 B1**
(45) Date of publication and mention of the grant of the patent: **27.04.2011**
(21) Application number: 05774237.1
(22) Date of filing: 29.07.2005
(51) Int. Cl.: A61K 31/7088, A61K 39/39

(54) **ADJUVANT COMPRISING OLIGONUCLEOTIDE AND NON TOXIC LPS**
ADJUVANS MIT EINEM OLIGONUKLEOTID UND NICHT-TOXISCHEM LPS
ADJUVANT COMPRENANT UN OLIGONUCLEOTIDE ET DES LPS NON TOXIQUES

(30) Priority: 10.05.2005 KR 20050038771
(43) Date of publication of application: 23.01.2008
(73) Proprietor: Eyegene Inc., 120-113 Seoul (KR); Lee, Na-Gyong, 577, Gwangjang-dong, Gwangjin-gu Seoul 143-210 (KR)
(72) Inventor: AHN, Bo-Young, Seoul 120-132 (KR); YOO, Won-Il, Sungnam-si, Gyeonggi-do 463-020 (KR); CHO, Yang-Je, Seoul 140-748 (KR); LEE, Na-Gyong, Gwangjin-gu, Seoul 143-210 (KR)
(74) Representative: Tombling, Adrian George
(86) International application number: PCT/KR2005/002490
(87) International publication number: WO 2006/121232

(56) References cited:
- WO-A-2004/039413
- WO-A1-2004/039413
- YI ET AL: "CpG DNA synergize for tumor necrosis factor-a production through activation of NF-kB" INTERNATIONAL IMMUNOLOGY, OXFORD UNIVERSITY PRESS, GB, vol. 13, no. 11, 1 November 2001 (2001-11-01), pages 1391-1404, XP002986604 ISSN: 0953-8178
- GAO J J ET AL: "CUTTING EDGE: BACTERIAL DNA AND LPS ACT IN SYNERGY IN INDUCING NITRIC OXIDE PRODUCTION IN RAW 264.7 MACROPHAGES" JOURNAL OF IMMUNOLOGY, AMERICAN ASSOCIATION OF IMMUNOLOGISTS, US, vol. 163, no. 8, 15 October 1999 (1999-10-15), pages 4095-4099, XP001206756 ISSN: 0022-1767
- GAO J J ET AL: "BACTERIAL DNA AND LIPOPOLYSACCHARIDE INDUCE SYNERGISTIC PRODUCTION OF TNF-ALPHA THROUGH A POST-TRANSCRIPTIONAL MECHANISM" JOURNAL OF IMMUNOLOGY, AMERICAN ASSOCIATION OF IMMUNOLOGISTS, US, vol. 166, no. 11, 1 June 2001 (2001-06-01), pages 6855-6860, XP001206755 ISSN: 0022-1767
- HUANG HONG ET AL: "[The synergistic effects of lipopolysaccharide, bacterial lipoprotein and bacterial DNA on mouse alveolar macrophage activation]" ZHONGHUA YI XUE ZA ZHI 8 JUN 2005, vol. 85, no. 21, 8 June 2005 (2005-06-08), pages 1468-1472, XP002530641 ISSN: 0376-2491
- YI ET AL.: 'Lipopolyaccharide and CpG and DNA synergize for tumor necrosis factor-a production through activation of NF-kB' INTERNATIONAL IMMUNOLOGY vol. 13, no. 11, November 2001, pages 1391 - 1404, XP002986604
- HACKER ET AL.: 'Activation of the immune system by bacterial CpG-DNA' IMMUNOLOGY vol. 105, no. 3, March 2002, pages 245 - 251, XP002986603

<110> EYEGENE INC.
   LEE. NA GYONG
<120> Adjuvant comprising oligonucleotide and non-toxic LPS
<160> 3
<170> Kopatentln 1.71
<210> 1
   <211> 20
   <212> DNA
   <213> Artificial Sequence
<220>
   <223> ODN 1826.
<400> 1
   tccatgacgt tcctgacgtt 20
<210> 2
   <211> 24
   <212> DNA
   <213> Artificial Sequence
<220>
   <223> ODN 7909
<400> 2
   tcgtcgtttt gtcgttttgt cgtt 24
<210> 3
   <211> 24
   <212> DNA
   <213> Artificial Sequence
<220>
   <223> ODN nonCG
<400> 3
   ctggtctttc tggttttttt ctgg 24

## Description

### TECHNICAL FIELD

The present invention relates to an adjuvant using a lipopolysaccharide (LPS)-derived non-toxic high-molecular compounds (CIA05) and oligodeoxynucleotides (ODNs).

### BACKGROUND ART

Generally, one of the distinctive differences between mammalian and bacterial DNAs is the significant CpG suppression and the selective methylation of CpG dinucleotides at cytosine residues in the mammalian DNA. Recently, the researchers has proposed that CpG motifs present in the bacterial DNA rapidly activate polyclonal B cells to facilitate secretion of IgM, and the bacterial CpG motifs inhibit expression of c-myc mRNA and increase expression of myn, blc2 and bcl-XL mRNAs to protect the cells from being apoptosed in the B cells in which the cell cycles are stopped by anti-IgM antibodies and apoptosis is initiated. In another study, it was reported that a CpG motif directly activates B cells to facilitate secretion of IL-6 and IL-12 within a short time. Clinical trials of an adjuvant and a therapeutic agent for treatment of asthma using synthetic oligonucleotides including the CpG sequences have been in progress by the company CPG (U.S), based on the characteristics described above.

In the recent studies, it was, however, reported that cytosine methylation in the CpG dinucleotides is not associated with an anti-cancer effect, and it is also reported that an effect of stimulating immune reaction by bacterial DNA depends on its structural factor, etc.

However, it was reported that such a role of the unmethylated CpG is not necessary in DNA anti-cancer drugs, and methods which may be in place of this method remain to be developed.

Adjuvant compositions comprising non-methylated CG ODNs and non-toxic LPS for stimulating immune response are disclosed in WO 2004/039413 and the synergistic effect of the combination of such ODNs with LPS is describes by Yi et al (International Immunology, 2001, vol. 13, pages 1391-1404), Gao et al (The journal of Immunology, 1999, vol. 163, pages 4095-4099) and Gao et al (The journal of Immunology, 2001, vol. 166, pages 6855-6860).

LPS is a typical thymus-independent antigen, and known to cause side effects such as inflammation, etc. by directly acting on B cells to induce non-specific immune reactions. But, it was seen that LPS can use its toxicity to kill cancer cells, and its subunit Lipid A especially shows an anti-cancer effect by inducing expression of the various transcription factors. But, LPS has a strong toxicity as a typical endotoxin. In addition, binding of a general LPS to DNA may cause a serious condition such as sepsis.

### DISCLOSURE OF INVENTION

Accordingly, the present invention is designed to solve the problems of the prior art, and therefore it is an object of the present invention to provide a material which is much safer and more effective than the conventional therapeutic agents and induces more specific immune reactions.

In order to accomplish the above object, the present invention provides an adjuvant composition comprising:
(a) oligodeoxynucleotides (ODNs) comprising CG motifs which are methylated at cytosine residues or ODNs not comprising a CG motif and
(b) bacterial LPS-derived non-toxic high-molecular material which has a molecular weight of 2,000 to 10,000 daltons and has degraded Lipid A.

In the present invention, it is not important whether or not an unmethylated CG is present in the ODNs, but the non-toxic compound has a molecular weight of about 2,000 to 10,000 daltons.

Also in the present invention, a content of the ODNs and the bacterial LPS-derived non-toxic compounds may be used if they are mixed at a minimum amount to show the effect of the present invention. Particularly, their efficiency is increased in the weight ratio of 500:1 to 1:500 in a dose-dependant manner, and the range is preferred, considering their non-toxicity, economical efficiency, etc.

Also, the two components are preferably mixed by shaking. The interaction of the CpG motif as described above mainly appears by inducing immunoactivation of T helper type 1 cells and activation ofNK cells.

Also, the bacterium, used in the present invention, is preferably Escherichia coli or mycobacteria, and more preferably Escherichia coli.

Also, the composition is preferably used as a vaccine adjuvant, and more preferably as an HBV vaccine adjuvant.

### BRIEF DESCRIPTION OF THE DRAWINGS

Other objects and aspects of the present invention will become apparent from the following description of embodiments with reference to the accompanying drawings in which:

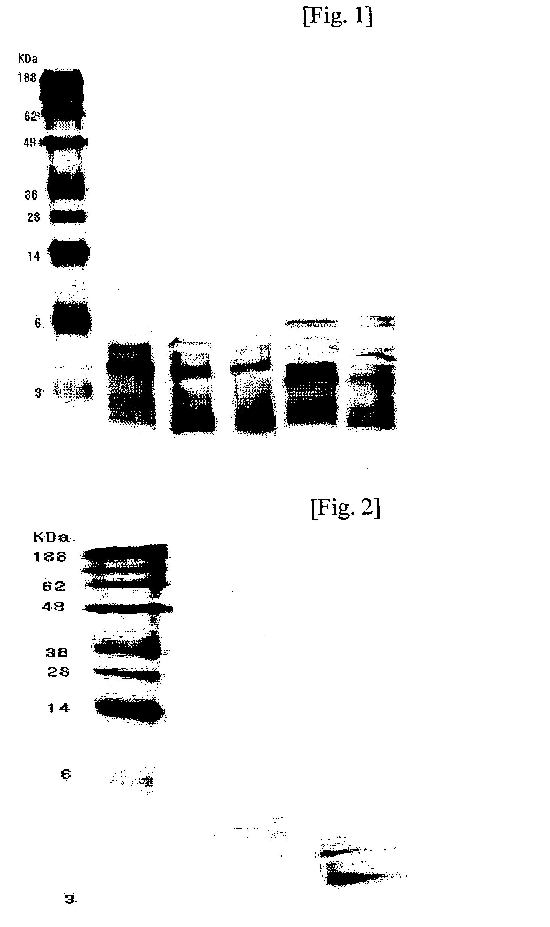

Fig. 1 is an electrophoretic diagram showing separated products of lipopolysaccharides from the outer membrane of *E. coli* cells. The diagram shows the separated products of the lipopolysaccharides in 5 batch experiments, respectively.
Fig. 2 is an electrophoretic diagram showing that Lipid A is degraded by alkaline treatment, and its size is reduced in the separated *E. coli* lipopolysaccharides, and therefore their toxicity is removed. In the diagram, lane 1 represents a marker, lane 2 represents separated products of lipopolysaccharides (CIA04), and lane 3 represents alkaline-treated non-toxic lipopolysaccharides (CIA05).

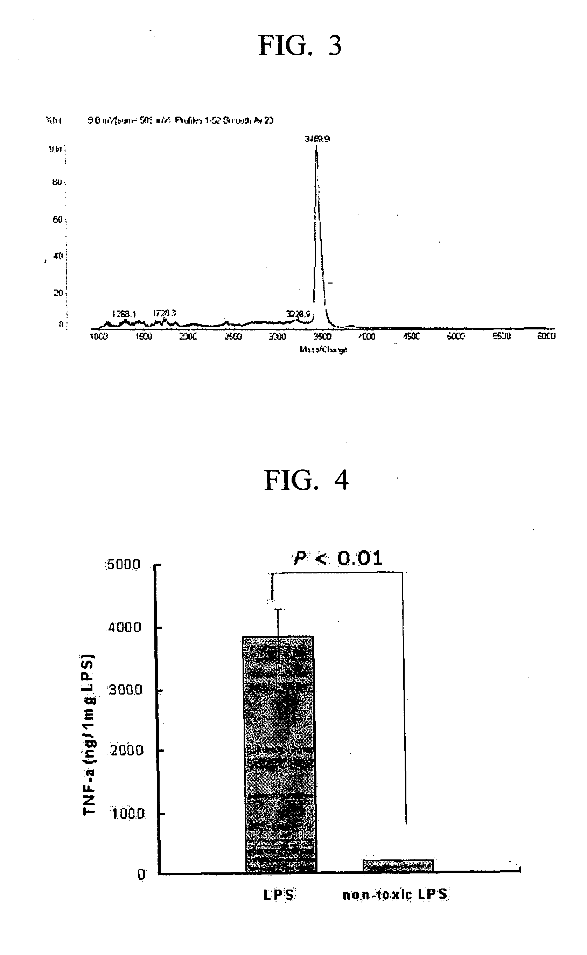

Fig. 3 is a diagram showing that a molecular weight of CIA04 is measured using a MALDI-MASS. The CIA04 is dissolved in distilled water at a concentration of 5 mg/mℓ, and used. Gentisic acid (2,5-dihydroxybenzoic acid, Sigma, G-5254) is used as a matrix. Axima-LNR V 2.3.5 (Mode Liner, Power: 106) from the company Shimadz is used as the MALDI-MASS.
Fig. 4 is a diagram showing that an amount of TNF-α secreted in THP-1 (Acute monocytic leukemia) is measured. The control lipopolysaccharide induces the THP-1 cells to secrete a large amount of TNF- α, while the non-toxic CIA05 induces the THP-1 cells to secrete an extremely low amount of TNF-α, indicating that inflammatory reaction by the toxicity of the lipopolysaccharide is reduced significantly.

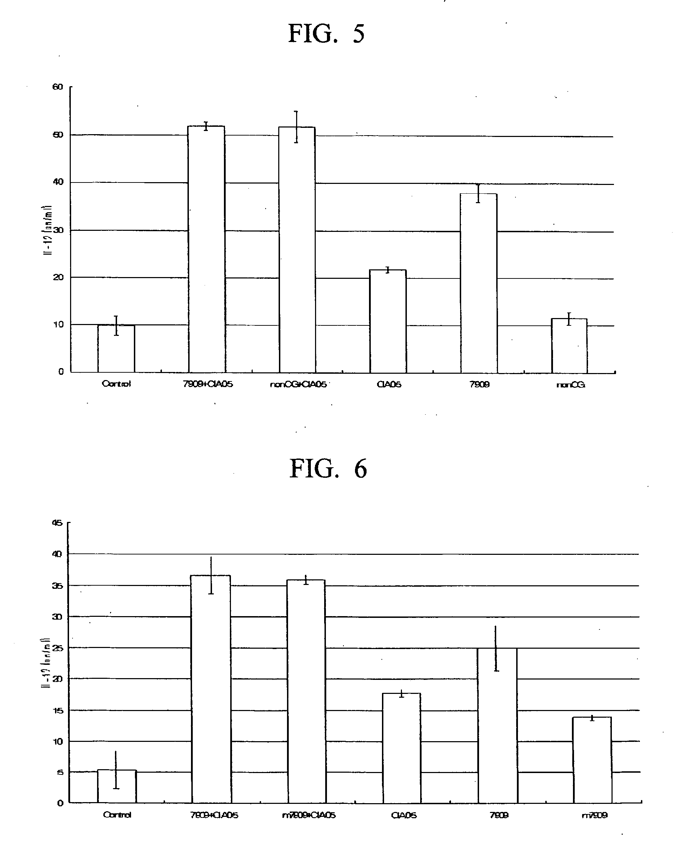

Fig. 5 is a diagram showing, from an amount of IL-12 expressed in human blood cells, that CIA05 has an effect of stimulating immune reaction regardless of whether or not a GC sequence is present in the oligodeoxynucleotides (ODNs).
Fig. 6 is a diagram showing, from an amount of IL-12 expressed in human blood cells, that improved DNA anti-cancer efficiency of CIA05 is not associated with the unmethylated CG by means of the CG methylation. Here, m7909 represents cytosine-methylated ODN 7909.

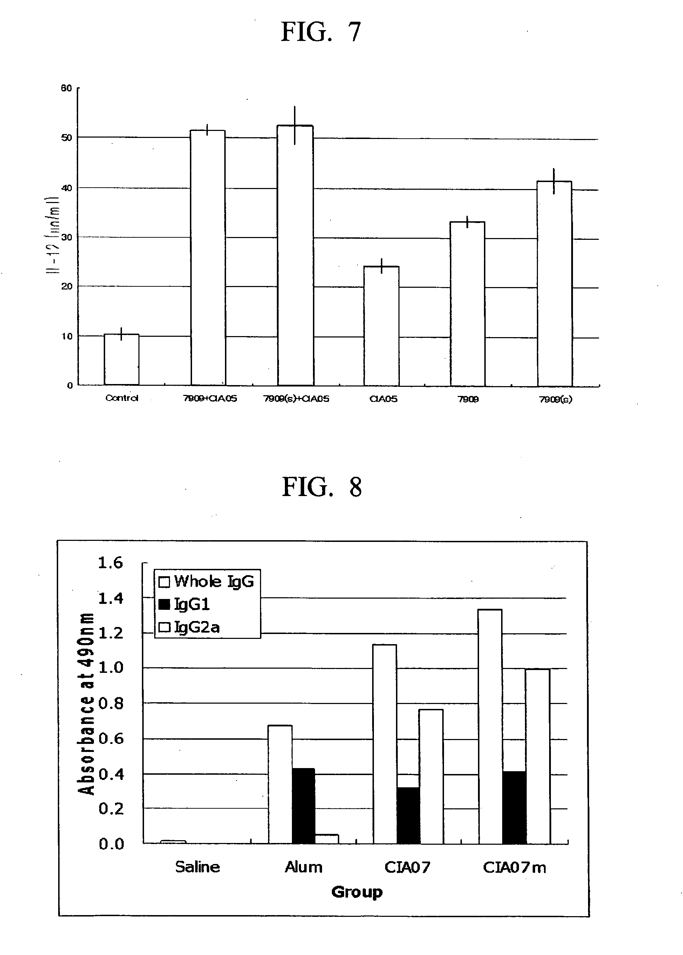

Fig. 7 is a diagram showing that immunity of the ODN having phosphorothioate is improved by CIA05. Here, 7909(s) represents the phosphorothioate form ODN 7909.
Fig. 8 is a graph showing an effect of the ODN by CIA05 as a vaccine adjuvant in a mouse model. Here, it shows that binding of CIA05 to the ODN play an important role, particularly in activation of the immune cells.

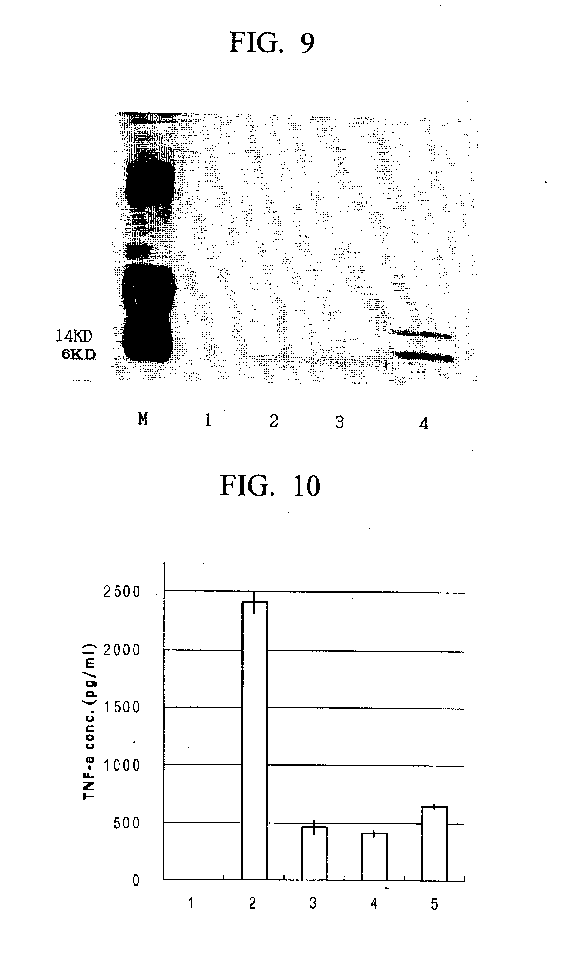

Fig. 9 is an electrophoretic diagram showing that major fractions having a low molecular weight, obtained from LPS lysate by a gel filtration using a sephacryl S-200HR (Pharmacia), are observed on SDS-PAGE. At this time, 14 % tris-glycine gel is used and silver-stained. Then, it was confirmed that Fractions 2 and 3 used in this experiment have a molecular weight of less than 10,000 daltons. The diagram shows the SDS-PAGE of LPS and its cleaved derivatives from *E. coli.* In the diagram, M represents a pre-stained marker, lane 1 represents a treated LPS (Fraction 1), lane 2 represents a treated LPS (Fraction 2), lane 3 represents a treated LPS (Fraction 3), and lane 4 represents untreated LPS (20 kD).
Fig. 10 is a graph showing, from a level of TNF- α secreted in human PBMCs, that toxicity of LPS is varied according to its size. In the graph, "1" represents saline, "2" represents LPS (20 kD Sigma L2880), "3" represents lysed LPS (5 kD to 10 kD), "4" represents CIA05 (3.5 kD), and "5" represents MPL (2 kD LPS, Sigma L6638).

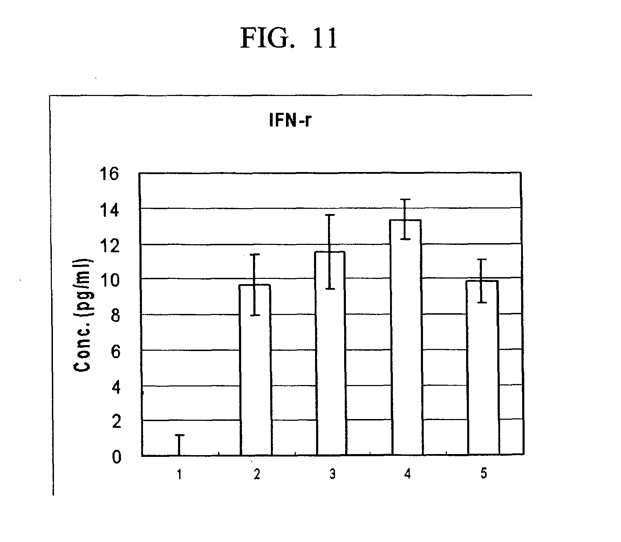

Fig. 11 is a graph showing, from a level of TNF- α secreted in the healthy male's venous blood, that an immune-enhancing effect of LPS is varied according to its size. In the graph, "1" represents saline, "2" represents LPS (20 kD Sigma L2880), "3" represents lysed LPS (5 kD to 10 kD), "4" represents CIA05 (3.5 kD), and "5" represents MPL (2 kD LPS, Sigma L6638).

### BEST MODES FOR CARRYING OUT THE INVENTION

Hereinafter, preferred embodiments of the present invention will be described in detail referring to the accompanying drawings.

The inventors designed a bacterial LPS-derived non-toxic high-molecular material (CIA05) as the adjuvant, and confirmed that the high-molecular material (CIA05) is effectively used as the adjuvant. Especially, an oligonucleotide shows no effect if it is used alone, but the oligonucleotide has the effect as described above if it is used in combination with the high-molecular material (CIA05).

Binding of DNA to general lipopolysaccharides allows the lipopolysaccharides to participate in various reactions, for example by functioning as a T cell-independent antigen in various sites of the immune system, and therefore their synergic effect may cause serious conditions such as sepsis. However, CIA05 show no specific toxicity even though it is used in combination with DNA.

The inventors screened a strain (*E. coli* EG0021) having a very short sugar chain of lipopolysaccharide from Escherichia coli living in the bowls of healthy humans and deposited the strain *E. coli* EG0021 to the Korean Culture Center of Microorganisms (KCCM) at 361-221 Hongje-dong, Seodaemun-gu, Seoul, on May 2, 2002, and its accession number was KCCM 10374. And, there was established a method for purifying lipopolysaccharides from the strain *E. coli* EG0021. Also, fatty acid was removed from the resultant very small LPS by alkaline treatment to obtain CIA05, which is very safe and shows an anti-cancer effect.

The following oligodeoxynucleotides (ODNs) were synthesized, which are commercially available from the company Genotech Co. Ltd.
ODN 1826 TCCATGACGTTCCTGACGTT (SEQ ID NO: 1; 20 mer)
ODN 7909 TCGTCGTTTTGTCGTTTTGTCGTT (SEQ ID NO: 2; 24 mer)
ODN 7909m TCmGTCmGTTTTGTCmGTTTTGTCmGTT (cytosine methylation)
ODN 7909s TCGTCGTTTTGTCGTTTTGTCGTT (phosphorothioate)
ODN nonCG CTGGTCTTTCTGGTTTTTTTCTGG (SEQ ID NO: 3; 24 mer)

It was confirmed that a mixture of the ODN and CIA05, prepared by the method, might show a more improved efficiency.

The present invention has been described in detail with reference to non-limiting embodiments of the invention.

### Example 1: Screening of Non-toxic Strains

### Screening and Finding of Mutant Escherichia Coli Strain with Very Short Lipopolysaccharides

The strain *E. coli* EG0021 having a very short sugar chain of lipopolysaccharides was found from Escherichia coli living in the bowls of healthy humans, and there was established a method for purifying the lipopolysaccharides from the strain *E. coli* EG0021.

A single colony of the *E. coli* obtained from the healthy male adults was cultured in a liquid medium, and then a selection procedure was repeated 5 times to obtain 50 *E. coli* strains. And, each colony was taken from the 50 selected strains on the plates, dissolved in 4 mℓ of 0.9 % saline, and then 1 mℓ of the resultant solutions were transferred into Eppendorf tubes and treated with 2µℓ of DNase 1, and then reacted at 37°C in an incubator for 1 hours. After treatment with DNase 1, lysates were treated with 50 µℓ of RNase (10 mg/mℓ), and then reacted at 37 °C in an incubator for 1 hours. Then, 100 µℓ of Proteinase K (20 mg/mℓ) was added thereto, and then reacted at 37 °C overnight. A human lymphocyte cell line differentiated with GM-CSF was treated with each LPS of the strains obtained by the procedure as described above, and a level of the secreted TNF- α was measured, and then a strain having the lowest level of TNF- α was selected (Table 1), and a molecular weight of the lipopolysaccharide was confirmed on an electrophoresis. It was confirmed that generic characteristics of the attenuated strain itself or its morphological Characteristics did not changed, but a ladder of the lipopolysaccharide having a molecular weight of 50,000 to 100,000 daltons is absent and the lipopolysaccharide having a molecular weight of 2,000 to 10,000 daltons is produced mainly when its lipopolysaccharides were isolated and electrophoresed on the SDS-PAGE (Fig. 1). Accordingly, this strain was named EG0021.

**Table 1**

| Levels of Secreted TNF-α in Lysate of *E. coli* Separated from the Bowls of Healthy Human | | | | | | | | | |
|---|---|---|---|---|---|---|---|---|---|
| No. | TNF-α (pg/1µℓ) | No. | TNF-α (pg/1 µℓ) | No. | TNF-α (pg/1µℓ) | No. | TNF-α (pg/1 µℓ) | No. | TNF-α (pg/1 µℓ) |
| EG0001 | > 100 | EG0011 | 39 | EG0021 | 0.1 | EG0031 | 21 | EG0041 | 65 |
| EG0002 | 12 | EG0012 | 64 | EG0022 | > 100 | EG0032 | 1.2 | EG0042 | 54 |
| EG0003 | 72 | EG0013 | 8.8 | EG0023 | > 100 | EG0033 | > 100 | EG0043 | > 100 |
| EG0004 | 85 | EG0014 | 9 | EG0024 | > 100 | EG0034 | > 100 | EG0044 | > 100 |
| EG0005 | 25 | EG0015 | 70 | EG0025 | 53 | EG0035 | 7 | EG0045 | 17 |
| EG0006 | 35 | EG0016 | > 100 | EG0026 | 12 | EG0036 | 87 | EG0046 | 2.1 |
| EG0007 | 71 | EG0017 | 6 | EG0027 | 4 | EG0037 | 0.7 | EG0047 | 3.5 |
| EG0008 | 28 | EG0018 | 11 | EG0028 | 76 | EG0038 | 39 | EG0048 | > 100 |
| EG0009 | 2 | EG0019 | 0.3 | EG0029 | 92 | EG0039 | 37 | EG0049 | > 100 |
| EG0010 | 13 | EG0020 | 80 | EG0030 | > 100 | EG0040 | 91 | EG0050 | > 100 |

### Example 2: CG methylation

In order to characterize functions of unmethylated CG in the oligonucleotide, the cytocine residues of CG sequences were selectively methylated with Sss I methyalse.

DNA methylation was carried out by mixing 1 unit of CpG methylase (M. Sss I; NEB M0226S) with 10 ug of ODN 7909, and then reacting each other at 37 °C for 12 hours. At this time, 160 µ M S-adenosylmethionine (SAM) was mixed as a methyl donor and reacted together. After the methylation was completed, the remaining salts and enzymes were, then, removed off using a DNA clean kit (CPG DPC60050) and a micropure EZ (Amicon 42529).

### Example 3: Purification of CIA 04 from Mutant E. coli

### Purification of Lipopolysaccharide from Mutant E. coli

The strain *E. coli* was prepared in the same manner as in the DNA separation.

The strain prepared thus was mixed with 2 × volumes of ethanol, centrifuged at 4,000 g to precipitate a pellet, and then 1.5 × volumes of acetone was added to the resultant pellet, mixed throughly and centrifuged at 4,000 g.

The equivalent amount of ethyl ether wad added to the resultant pellet, mixed throughly and centrifuged at 4,000 g. The cell pellet obtained by centrifugation was covered with an aluminum foil with holes in it, and dried, and the cell body was weighed, and then an extraction mixture (90 % Phenol : Chloroform : Petroleum ether = 2 : 5 : 8) was added at an amount of 7.5 mℓ, per 1 g of the dried weight. The resultant mixture was divided into glass centrifuge tubes and centrifuged at 25 °C and 3,000 rpm (1,200 g) for 20 minutes to obtain supernatant. The resultant supernatant was kept in a hood for 12 hours to precipitate the residues, divided into glass centrifuge tubes and centrifuged at 25 °C and 3,000 rpm (1,200 g) for 20 minutes to obtain lipopolysaccharides. The resultant lipopolysaccharides were dissolved in ethyl ether, and then the lipopolysaccharide solutions were transferred to Eppendorf tubes, dried in a hood, and their dried weights were measured using a chemical balance, and then ethanol was added to the dried lipopolysaccharide, which was stored for the future use. Ethanol was completely removed from the purified *E. coli* lipopolysaccharide stored in ethanol, and then an amount of KDO (2-keto-3-deoxyoctonate) in the lipopolysaccharides was measured, normalized as a standard to measure its concentration, and separated according to their molecular weight on the SDS-PAGE, and their molecular weight was confirmed using a silver staining method. It was confirmed that the lipopolysaccharide has a molecular weight of about 2,000 to about 10,000 daltons, which is very smaller than the general *E. coli* lipopolysaccharides (Fig. 2).

Meanwhile, Fig. 3 is a diagram showing that a molecular weight of CIA04 is measured using a MALDI-MASS. The CIA04 is dissolved in distilled water at a concentraion of 5 mg/mℓ, and used. Gentisic acid (2,5-dihydroxybenzoic acid, Sigma, G-5254) is used as a matrix. Axima-LNR V 2.3.5 (Mode Liner, Power: 106) from the company Shimadz is used as the MALDI-MASS. As seen from Fig. 3, it was revealed that the CIA04, as measured using the MALDI-MASS, has a molecular weight of about 3,500 daltons (Fig. 3).

### Example 4: Removal of Toxicity of Lipopolysaccharide Purified from Mutant E. coli

### Removal of Toxicity by Degradation of Lipid A in Lipopolysaccharide

The purified *E. coli* lipopolysaccharide was adjusted to a concentration of 3 mg/mℓ, and 0.2 N NaOH was mixed with the lipopolysaccharide at a mixing ratio of 1:1 (by volume), deacylated for 140 minutes while shaking at 60°C every 10 minutes, and then 1 N acetic acid was added at about 1/5 amount of the initial 0.2 N NaOH to titrate to pH 7.0. After titration of pH, the resultant mixture was precipitated by ethanol to obtain non-toxic lipopolysaccharide.

Concentration of the non-toxic lipopolysaccharide was measured using a KDO method, and the non-toxic lipopolysaccharide was compared with an untreated lipopolysaccharide on the SDS-PAGE, and then its molecular weight was confirmed using a silver staining method.

As a result of the staining, it was revealed that Lipid A of the lipopolysaccharide was degraded by the alkaline treatment, and therefore was smaller than the untreated lipopolysaccharide (CIA04) (Fig. 2).

### Confirmation on Removal of Toxicity from Non-toxic Lipopolysaccharide

A secretion test of inflammatory proteins and a pyrogen test were conducted in order to confirm that toxicity of the LPS is reduced to at least 1/1,000 times in the method for selecting strains that synthesize smaller LPSs, and that its toxicity is further reduced using the alkaline treatment.

### - Secretion of Inflammatory Protein

A level of TNF- α secreted in THP-1 (Acute monocytic leukemia) was measured. It was seen that a large amount of TNF- α was secreted by the control lipopolysaccharide, while a very small amount of TNF- α was secreted by the non-toxic LPS (CIA05), indicating that the inflammatory reactions by its toxicity was significantly relieved (Fig. 4).

### - Pyrogen Test

3 rabbits were vaccinated to check a change of temperature in their recta, as follows. A vaccine was intravenously injected into the rabbit ears at an amount of 0.2 µg/l mℓ per 1 kg of a rabbit, and then each thermometer was inserted into their recta to check their abnormal changes of temperature. Rabbits with body weights of at least 1.5 kg was used in this experiment. The rabbits used in the test should be re-used after at least 3 days. A thermometer, which can measure temperature with a 0.1 °C resolution, was used to measure their body temperatures. Syringes and needles, previously sterilized by heating at 250 °C for at least 30 minutes, were used. Animals were fed only with water at a period from 16 hours before their use until the experiment was completed. Fixation of animals was conducted as moderate as it can be.

Measurement of the body temperature was carried out by inserting the thermometer into a rectum at the constant depth of 60 mm to 90 mm, and checking its temperature after a predetermined time. The temperature measured before injection of the vaccine was used as a control temperature. The sample pre-warmed to about 37 °C were intravenously injected into the rabbit ears within 15 minutes after the control temperature was measured. The body temperature was checked very 3 hours, at leased every 1 hour after injection. A difference of the measured temperatures and the control body temperature was calculated, and the difference was referred to as a difference of body temperature. And, the maximum difference of body temperature was considered as an exothermic reaction of the test animal. 3 animals of a specimen were used in this experiment.

If a sum of the temperatures measured in the 3 animals is 1.3 °C or less, a pyrogen test is considered to be "negative", while if it is 2.5 °C or more, a pyrogen test is considered to be "positive". This experiment was repeated 3 times, and the vaccine was suitable for this experiment since the pyrogen test was proven to be negative. The result is listed in the following Table 2.

**Table 2**

| Times | No. | Before Injection (Measured 3 Times) | | | After Injection (Hour, Intervals: 30 Minutes) | | | | | | Increased Body Temp. | Sun of Increased Body Temp. | Result | Reference Temp. |
|---|---|---|---|---|---|---|---|---|---|---|---|---|---|---|
| | | 1 | 2 | 3 | 0.5 | 1 | 1.5 | 2 | 2.5 | 3 | | | | |
| 1 | Rabbit 1 | 39.1 | 39.2 | 39.2 | 39.4 | 39.3 | 39.2 | 39.2 | 39.1 | 39.1 | 0.2 | 0.8 | Passed | Less than 1.3 °C |
| | Rabbit 2 | 39 | 39.1 | 39.3 | 39 | 39.2 | 39.5 | 39.2 | 39.1 | 39.3 | 0.4 | | | |
| | Rabbit 3 | 39.4 | 39.2 | 39.2 | 39.3 | 39.5 | 39.3 | 39.5 | 39.3 | 39.4 | 0.2 | | | |
| 2 | Rabbit 1 | 39 | 39.3 | 39.1 | 39.4 | 39.2 | 39.3 | 39.1 | 39.2 | 39 | 0.4 | 1.7 | Passed | Less than 3.0 °C |
| | Rabbit 2 | 39.4 | 39.2 | 39.2 | 39.1 | 39.1 | 39.3 | 39.1 | 39.2 | 39.2 | 0.3 | | | |
| | Rabbit 3 | 39.3 | 39.3 | 39.2 | 39.4 | 39.4 | 39.4 | 39.4 | 39.4 | 39.3 | 0.2 | | | |
| 3 | Rabbit 1 | 39.2 | 39.2 | 39.1 | 39.2 | 39.2 | 39 | 39.2 | 39.1 | 39.1 | 0.2 | 2.5 | Passed | Less than 5.0 °C |
| | Rabbit 2 | 39.1 | 39.5 | 39 | 39 | 39.1 | 39.2 | 39.1 | 39.3 | 39.2 | 0.4 | | | |
| | Rabbit 3 | 39.2 | 39.3 | 39.2 | 39.3 | 39.2 | 39.3 | 39.2 | 39.4 | 39.3 | 0.2 | | | |

### Example 5: Mix of Oligodeoxynucleotide (ODN) and Non-toxic Lipopolysaccharide-Derived Polysaccharide (CIA05) and Their Activity

### Efficiency Test of Mixture of ODN and CIA05

Venous blood was aseptically taken from healthy adult males, and put into a vacuum tube including an anti-coagulant heparin. The resultant whole blood was mixed with an RPMI 1640 medium (2 mM L-glutamine, 1 mM Sodium pyruvate, 80 µg/mℓ of gentamycin) at a mixing ratio of 1:1. 20 µℓ of CIA07 (50 µg of CIA02 + 1 µg or 500 ng of CIA05, 100 ng) or 20 µℓ of HBSS were added to 1 mℓ of the whole blood mixed with the medium together, and then incubated at 37 °C in a 5 % CO₂ incubator for 24 hours. Then, a culture supernatant was collected to measure levels of secreted TNF-α (R&D system, DY210) and secreted IL-12 p40 (R&D system, DY1240) using a commercially-available ELISA kit. The results are shown in Figs. 4 to 7.

From the result as described above, it was revealed that CIA05 showed an immune-stimulating effect regardless of whether or not a GC sequence is present in the oligodeoxynucleotide (ODN). In particular, it was revealed that the unmethylated CG-free ODN (nonCG) showed a similar immune-stimulating effect to that of saline used as the control if it was used alone, but showed a strong immune-stimulating effect if it was used in combination with CIA05 (nonCG + CIA05) (Fig. 5). Such a synergic effect was clearly confirmed by the cytosine methylation of the GC sequence in the ODN. That is, the ODN (m7909) methylated at a cytosine residue of a GC sequence of 7909 ODN (7909) showed a low immune-stimulating effect if it was used alone, but showed the nearly same strong immune-stimulating effect as in the case of the mixture of the 7909 ODN and the CIA05 (7909 + CIA05) if it was used in combination with CIA05 (m7909 +CIA05). Accordingly, it was confirmed that the improved DNA anti-cancer efficacy of the CIA05 was not correlated with the unmethylated CG (Fig. 6). Also, it was revealed that the ODN including phosphorothioate also showed an improved immunoefficiency. 7909(s) is a oligodeoxynucleotide in which a diester bond is substituted with phosphorothioate in the 7909 ODN (Fig. 7).

### Measurement of Adjuvant Effect of CIA using Mouse Model System

- Yeast recombinant HBs antigen was used at a concentration of 219 µg/mℓ as the antigen. Alum hydroxide was used as the adjuvant in the control group, and CIA05 was mixed with DNA methylated at the base C of the CpG (CIA07m) and general bacterial DNA (CIA07) at a mixing ratio of 1:100 and used as the adjuvant in the experimental group.

The used animal is an ICR mouse, and each group was grouped into 6 mice, and intramuscularly injected once every a week (3 times). The negative control group was injected with 0.1 mℓ of saline injection per mouse, and the positive control group was injected with 2 µg of HBs Ag + 50 µg of the alum, dissolved in 0.1 mℓ of saline for 2 hours. The experimental group was injected with 2 µg of HBs Ag and 50 µg of CIA07 or CIA07m. Finally, after 7 day of injection, whole blood was collected and centrifuged to obtain blood serum.

A level of IgG against HBs antigen in the blood serum was measured using ELISA. As a result, it was seen that CIA07 and CIA07m showed an excellent effect on antibody production, compared to the case where the alum was used alone, and a level of IgG2 was especially increased, indicating that they may considerably contribute to improving their cell-mediated immunity, which is important for development of virus or cancer vaccines (Fig. 8).

### Example 6: Toxicity and Efficiency of LPS according to its Size

### LPS Lysate with Molecular Weight of 2,000 to 10,000 Da Obtained by Lysing General LPS

A procedure where *E. coli* LPS (055:B5, Sigma) is sonicated at 150 J for 2 minutes, and then kept for 1 minute was repeated 20 times. LPS lysate obtained thus was gel-filtered using a sephacryl S-200HR (Pharmacia).

Fig. 9 is an electrophoretic diagram showing that major fractions having a low molecular weight, obtained from LPS lysate by a gel filtration using a sephacryl S-200HR (Pharmacia), are observed on SDS-PAGE. At this time, 14 % tris-glycine gel is used and silver-stained. Then, it was confirmed that Fractions 2 and 3 used in this experiment have a molecular weight of less than 10,000 daltons. The diagram shows the SDS-PAGE of LPS and its cleaved derivatives from *E. coli.* In the diagram, M represents a pre-stained marker, lane 1 represents a treated LPS (Fraction 1), lane 2 represents a treated LPS (Fraction 2), lane 3 represents a treated LPS (Fraction 3), and lane 4 represents untreated LPS (20 kD). As seen in Fig. 9, the major fractions having a low molecular weight were confirmed on SDS-PAGE.

### Toxicity Test of LPS Lysate

Human PBMCs obtained from the healthy males were put into a 24 well tissue culture plate, and RPMI 1640 + 10 % FBS was added at a concentration of 5 X 10⁵ per 1 mℓ/well. The resultant mixture was treated with BSS or test materials, incubated for 12 hours, treated with 100 µℓ of BSS (Balanced salt solution) and 1 ug/100 µℓ of LPS, and then a level of TNF- α secreted by PBMCs was quantified using ELISA (R&D system, DY210).

Fig. 10 is a graph showing, from a level of TNF- α secreted in human PBMCs, that toxicity of LPS is varied according to its size. In the graph, "1" represents saline, "2" represents LPS (20 kD Sigma L2880), "3" represents lysed LPS (5 kD to 10 kD), "4" represents CIA05 (3.5 kD), and "5" represents MPL (2 kD LPS, Sigma L6638). As seen in Fig. 10, it was revealed that LPS with the low molecular weight, for example CIA05 of Lane 3, etc., showed a low toxicity.

### Immune-Enhancing Test on LPS Lysate

Whole blood, aseptically taken from healthy adult males and put into a vacuum tube including an anti-coagulant heparin, was mixed with an RPMI 1640 medium at a mixing ratio of 1:1. 1 mℓ of the whole blood mixed with the medium was added to a 24 well plate, respectively, treated with LPS, incubated at 37 °C in a 5 % CO₂ incubator for 12 hours, centrifuged to obtain supernatant, and then a level of IFN-γ was measured from the resultant supernatant using an ELISA kit (R&D system IFN-γ ; DY285).

Fig. 11 is a graph showing, from a level of TNF- α secreted in the healthy male's venous blood, that an immune-enhancing effect of LPS is varied according to its size. In the graph, "1" represents saline, "2" represents LPS (20 kD Sigma L2880), "3" represents lysed LPS (5 kD to 10 kD), "4" represents CIA05 (3.5 kD), and "5" represents MPL (2 kD LPS, Sigma L6638). As seen in Fig. 11, it was revealed that CIA05 of Lane 4 of the present invention showed an excellent immune-enhancing effect.

### INDUSTRIAL APPLICABILITY

As described abode, the bacteria-derived material (CIA05) of the present invention is significantly effective when compared to the case where the conventional oligodeoxynucleotide is used alone, and also may induce a specific immune reaction. Accordingly, the *E. coli*-derived adjuvant of the present invention may have a high industrial value.
## Claims

1. An adjuvant composition comprising:
(a) oligodeoxynucleotides (ODNs) comprising CG motifs which are methylated at cytosine residues, or ODNs not comprising a CG motif; and
(b) bacterial LPS-derived non-toxic high-molecular material which has a molecular weight of 2,000 to 10,000 daltons and has degraded Lipid A.

2. The adjuvant composition according to claim 1, wherein the oligodeoxynucleotides are at least 20-mers in size.

3. The adjuvant composition according to claim 1 or claim 2, wherein the oligodeoxynucleotides comprise the nucleotide sequence as set forth in SEQ ID NO: 2 in which CG motifs are methylated at cytosine residues or the nucleotide sequence as set forth in SEQ ID NO: 3.

4. The adjuvant composition according to claim 1, wherein a weight ratio of the oligodeoxynucleotides to the LPS-derived non-toxic high-molecular material ranges from 500:1 to 1:500.

5. The adjuvant composition according to claim 1, wherein the bacteria is selected from the group consisting of Escherichia coli and mycobacteria.

6. The adjuvant composition according to claim 1, wherein the component (a) and the component (b) are mixed by shaking.

7. Use of the adjuvant composition according to claim 1 as a vaccine adjuvant.

8. Use of the adjuvant composition according to claim 1 as an HBV vaccine adjuvant.

## Patentansprüche

1. Adjuvanszusammensetzung, umfassend:
(a) Oligodeoxynukleotide (ODN), die CG-Motive, welche an Cytosinresten methyliert sind, umfassen, oder ODN, die kein CG-Motiv umfassen; und
(b) bakterielles LPS-deriviertes nichttoxisches hochmolekulares Material, das ein Molekulargewicht von 2.000 bis 10.000 Dalton aufweist und degradiertes Lipid A aufweist.

2. Adjuvanszusammensetzung gemäß Anspruch 1, wobei die Oligodeoxynukleotide wenigstens 20-Mer-Größe haben.

3. Adjuvanszusammensetzung gemäß Anspruch 1 oder Anspruch 2, wobei die Oligodeoxynukleotide die in SEQ ID NO: 2 angegebene Nukleotidsequenz, bei welcher CG-Motive an Cytosinresten methyliert sind, oder die in SEQ ID NO: 3 angegebene Nukleotidsequenz umfassen.

4. Adjuvanszusammensetzung gemäß Anspruch 1, wobei ein Gewichtsverhältnis der Oligodeoxynukleotide zu dem LPS-derivierten nichttoxischen hochmolekularen Material im Bereich von 500: bis 1 :500 liegt.

5. Adjuvanszusammensetzung gemäß Anspruch 1, wobei die Bakterien aus der Gruppe ausgewählt sind, die aus Escherichia coli und Mykobakterien besteht.

6. Adjuvanszusammensetzung gemäß Anspruch 1, wobei die Komponente (a) und die Komponente (b) durch Schütteln gemischt sind.

7. Verwendung der Adjuvanszusammensetzung gemäß Anspruch 1 als Impfstoffadjuvans.

8. Verwendung der Adjuvanszusammensetzung gemäß Anspruch 1 als HBV-Impfstoffadjuvans.

## Revendications

1. Composition adjuvante comprenant :
(a) des oligodésoxynucléotides (ODN) comprenant des motifs CG qui sont méthylés au niveau de résidus de cytosine, ou des ODN ne comprenant pas de motif CG ; et
(b) un matériau non toxique à poids moléculaire élevé dérivé du LPS bactérien ayant un poids moléculaire de 2000 à 10 000 daltons et le lipide A dégradé.

2. Composition adjuvante selon la revendication 1, dans laquelle les oligodésoxynucléotides font au moins 20-mères.

3. Composition adjuvante selon la revendication 1 ou la revendication 2, dans laquelle les oligodésoxynucléotides comprennent la séquence nucléotidique telle qu'exposée dans le n° d'identification de SEQ : 2, dans laquelle les motifs CG sont méthylés au niveau de résidus de cytosine, ou la séquence nucléotidique telle qu'exposée dans le n° d'identification de SEQ : 3.

4. Composition adjuvante selon la revendication 1, dans laquelle un rapport pondéral des oligodésoxynucléotides par rapport au matériau non toxique à poids moléculaire élevé dérivé du LPS va de 500:1 à 1:500.

5. Composition adjuvante selon la revendication 1, dans laquelle la bactérie est sélectionnée parmi le groupe composé d'Escherichia coli et des mycobactéries.

6. Composition adjuvante selon la revendication 1, dans laquelle le composant (a) et le composant (b) sont mélangés par agitation.

7. Utilisation de la composition adjuvante selon la revendication 1 comme adjuvant de vaccin.

8. Utilisation de la composition adjuvante selon la revendication 1 comme adjuvant de vaccin contre le VHB.
